# NOUVEAU FASCICULE DE BREVET EUROPEEN

(11) **EP 1 084 694 B2**
(45) Date de publication et mention de la décision concernant l'opposition: **17.12.2008**
(45) Mention de la délivrance du brevet: 03.12.2003
(21) Numéro de dépôt: 00402373.5
(22) Date de dépôt: 28.08.2000
(51) Int. Cl.: A61Q 5/06, A61K 8/89

(54) **Composition cosmétique comprenant au moins un copolymère silicone/acrylate et au moins un agent de conditionnement**
Kosmetische Zusammensetzung die mindestens ein Silikon/Acrylat Copolymer und mindestens ein Konditionierungsmittel enthält
Cosmetic composition containing at least a silicone/acrylate copolymer and at least a conditioning agent

(30) Priorité: 16.09.1999 FR 9911590
(43) Date de publication de la demande: 21.03.2001
(60) Demande divisionnaire: 03290488.0
(73) Titulaire: L'ORÉAL, 75008 Paris (FR)
(72) Inventeur: Dupuis, Christine, 75018 Paris (FR)
(74) Mandataire: Dossmann, Gérard

(56) Documents cités:
- EP-A- 0 408 311
- EP-A- 0 749 747
- WO-A-01/13884
- WO-A-56/65337
- WO-A-56/67771
- WO-A-99/04750
- WO-A1-99/09939
- WO-A2-01/13884
- 'Fiedler Encyclopedia of Excipients', vol. I, 2002, EDITIO CANTOR VERLAG, AULENDORF, ISBN 3871932310 pages 580 - 581
- 'Wacker-Belsil ADM 6057 E Version 2.0' WACKER-BELSIL 21 Août 2003, MUNICH, pages 1 - 2

## Description

L'invention a pour objet une composition cosmétique, comprenant au moins un copolymère silicone/acrylate particulier et au moins un agent de conditionnement. Elle vise également un procédé cosmétique, en particulier un procédé de fixation et/ou de maintien de la coiffure mettant en oeuvre ladite composition ainsi que l'utilisation de cette composition pour la fabrication d'une formulation cosmétique, destinée notamment au maintien et/ou à la mise en forme de la coiffure.

Parmi les produits capillaires, notamment ceux destinés à la mise en forme et/ou au maintien de la coiffure les plus répandus sur le marché de la cosmétique, on distingue les compositions à pulvériser essentiellement constituées d'une solution le plus souvent alcoolique ou aqueuse et d'un ou plusieurs matériaux.

Lorsque les compositions sont destinées à la fixation et/ou au maintien de la coiffure, ces matériaux sont généralement des résines polymères, dont la fonction est de former des soudures entre les cheveux, appelés encore matériaux fixants, en mélange avec divers adjuvants cosmétiques. Ces compositions sont généralement conditionnées soit dans un récipient aérosol approprié mis sous pression à l'aide d'un propulseur, soit dans un flacon pompe.

On connaît également les gels ou les mousses capillaires qui sont généralement appliqués sur les cheveux mouillés avant de faire un brushing ou une mise en plis. Pour mettre en forme et fixer la coiffure, on effectue ensuite un brushing ou un séchage. Ces gels ou mousses peuvent également contenir des résines polymères.

Toutefois, ces compositions capillaires présentent souvent l'inconvénient d'altérer les propriétés cosmétiques des cheveux. Ainsi, les cheveux peuvent devenir rêches, difficiles à démêler, perdre leur toucher et leur aspect agréables ou encore manquer de corps. On recherche donc des compositions coiffantes procurant de bonnes propriétés cosmétiques, notamment en terme de démêlage, de douceur et de toucher.

En outre, ces compositions capillaires présentent également l'inconvénient majeur de donner lieu à une effet de poudrage. Au sens de la présente invention, on entend par « poudrage », l'aptitude du matériau obtenu par séchage de la composition capillaire à former une poudre après son application sur les cheveux. Bien entendu, la poudre tombe sur les épaules ou les vêtements de l'utilisateur, ou encore elle accroche au peigne ou à la brosse, et ceci est préjudiciable.

Il existe donc un besoin de trouver des compositions cosmétiques, notamment pour le coiffage, qui ne présentent pas l'ensemble des inconvénients indiqués ci-dessus, et qui en particulier fixent bien la coiffure, tout en procurant de bonnes propriétés cosmétiques, ceci sans effet de poudrage.

De manière surprenante et inattendue, la Demanderesse a découvert que lorsque l'on associe des copolymères silicone/acrylate particuliers avec certains agents de conditionnement, il est possible d'obtenir des compositions cosmétiques répondant aux exigences exprimées ci-dessus.

L'invention a pour objet une composition cosmétique comprenant, dans un milieu cosmétiquement acceptable constitué par des alcools en C₁-C₄ ou par des mélanges eau-alcools en C₁-C₄, au moins un copolymère silicone/acrylate et au moins un agent de conditionnement,
- le copolymère silicone/acrylate étant obtenu par polymérisation radicalaire d'au moins un monomère éthyléniquement insaturé (a) en présence d'au moins un dérivé siliconé (b) comprenant des groupements oxyalkylénés, et
- l'agent de conditionnement est une silicone non volatile comprenant des groupements aminés substitués ou non, directement fixés sur la chaîne siloxanique ou fixés par l'intermédiaire d'un radical hydrocarboné.

Un autre objet de l'invention concerne un procédé cosmétique capillaire, en particulier un procédé de fixation et/ou de maintien de la coiffure mettant en oeuvre ladite composition.

Encore un autre objet de l'invention concerne l'utilisation de cette composition pour la fabrication d'une formulation cosmétique capillaire, destinée notamment au maintien et/ou à la mise en forme de la coiffure.

Les copolymères silicone/acrylate particulièrement visés par la présente invention sont ceux décrits dans la demande internationale W099/04750. En particulier, on préfère le copolymère commercialisé par BASF sous la dénomination Luviflex Silk. Ce polymère un copolymère greffé acrylate de tertiobutyle/ acide méthacrylique et silicone copolyol.

De préférence, le monomère (a) répond à la formule (Iₐ) : 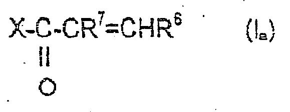 dans laquelle :
- X est choisi dans le groupe comprenant OH, OM, OR⁸, NH₂, NHR⁸, N(R⁸)₂ ;
- M est un cation choisi parmi Na⁺, K⁺, Mg⁺⁺, NH⁴⁺, alkylammonium, dialkylammonium, trialkylammonium, et tétraalkylammonium ;
- les radicaux R⁸ peuvent être identiques ou différents et sont choisis dans le groupe comprenant l'hydrogène, les groupements alkyls linéaires ou ramifiés en C₁ à C₄₀, les groupements alkyls en C₁ à C₄₀ mono- ou polyhydroxylés, éventuellement substitués par un ou plusieurs groupement(s) alcoxy, amino ou carboxy, les groupements polyéthers hydroxylés, les groupements N,N-diméthylaminoéthyl, 2-hydroxyéthyl, 2-méthoxyéthyl, 2-éthoxyéthyl, hydroxypropyl, méthoxypropyl et éthoxypropyl ;
- R⁷ et R⁶ sont choisis indépendamment l'un de l'autre dans le groupe comprenant l'hydrogène, les groupements alkyls linéaires ou ramifiés en C₁ à C₈, méthoxy, éthoxy, 2-hydroxyéthoxy, 2-méthoxyéthoxy et 2-éthoxyéthyl, les groupements CN, COOH et COOM.

En particulier, les monomères (a) de formule (la) sont l'acide acrylique et ses sels, esters ou amides. Les esters peuvent être dérivés d'alkyles linéaires en C₁ à C₄₀, d'alkyles ramifiés en C₃ à C₄₀ ou d'alcools carboxyliques en C₃ à C₄₀, d'alcools polyfonctionnels ayant 2 à 8 hydroxyles, tel que l'éthylène glycol, l'hexylène glycol, le glycérol et le 1,2,6-hexanetriol, d'aminoalcools ou d'éthers d'alcools tels que le méthoxyméthanol et l'éthoxyéthanol ou les polyalkylènes glycols.

Les monomères (a) de formule (la) peuvent également être choisis parmi les N,N-dialkylaminoalkyl acrylates et méthacrylates et N-dialkylaminoalkylacryl- et -méthacrylamides, le groupement amide pouvant être non substitué, N-aklyl- ou N-alkylamino-monosubstitué ou N,N-dialkylamino-disubstitué, les groupes alkyles ou alkylamino étant dérivés d'unités carboxyliques linéaires en C₁ à C₄₀ ou ramifiées en C₃ à C₄₀.

Les monomères (a) de formule (la) pouvant être utilisés peuvent aussi être des composés substitués dérivés de l'acide acrylique ou ses sels, esters ou amides. On peut citer par exemple les acides méthacrylique, éthacrylique et 3-cyanoacrylique.

D'autres monomères (a) convenant particulièrement bien sont les esters de vinyle et d'allyle en C₁ à C₄₀, les acides carboxyliques, linéaires en C₃ à C₄₀, ou carboxycycliques en C₃ à C₄₀, les halides de vinyle ou d'allyle, les vinyllactames, de préférence la vinylpyrrolidone et le vinylcaprolactame, les composés hétérocycles substitués par des groupement vinyles ou allyles, de préférence la vinylpyridine, la vinyloxazoline et l'allylpyridine, les N-vinylimidazoles, les diallylamines, le chlorure de vinylidène, les composés insaturés carbonés, comme le styrène ou l'isoprène, les dérivés quatemisés par l'épichlorhydrine de l'acide acrylique ou méthacrylique.

Comme autre monomère (a), on peut enfin citer les N-vinylimidazoles, les diallylamines, le chlorure de vinylidène, les composés insaturés carbonés comme le styrène et l'isoprène, les dérivés quaternisés par l'épichlorohydrine de l'acide acrylique ou méthacrylique.

On préfère tout particulièrement, comme monomère (a) l'acide acrylique, méthacrylique et éthylacrylique ; le méthyl, éthyl, propyl, n-butyl, isobutyl, t-butyl, 2-éthylhexyl et décyl acrylate ; le méthyl, éthyl; propyl, n-butyl, isobutyl, t-butyl, 2-éthylhexyl et décyl méthacrylate ; le méthyl, éthyl, propyl, n-butyl, isobutyl, t-butyl, 2-éthylhexyl et décyl éthacrylate ; le 2,3-dihydroxypropyl acrylate ; le 2,3-dihydroxypropyl méthacrylate ; le 2-dihydroxyéthyl acrylate ; l'hydroxypropyl acrylate le 2-hydroxyéthyl méthacrylate ; le 2-hydroxyéthyl éthacrylate ; le 2-méthoxyéthyl acrylate ; le 2-éthoxyéthyl méthacrylate ; le 2-éthoxyéthyl éthacrylate ; l'hydroxypropyl méthacrylate ; le glycéryl monoacrylate ; le glycéryl monométhacrylate ; les polyalkylènes glycols( méth-) acrylate, les acides sulfoniques insaturés, l'acrylamide, la méthacrylamide, l'éthacrylamide, la N,N-diméthylacrylamide, la N-éthylacrylamide, la N-éthylméthacrylamide, 1-vinylimidazole, N,N-diméthylaminoéthyl(méth-)acrylate, l'acide maléique, l'acide fumarique, l'anhydride maléique et ses monoesters, l'acide crotonique, l'acide itaconique, les éthers de vinyle, le vinylformamide, la vinylamine, la vinylpyridine, la vinylimidazole, le vinylfurane, le styrène, le sulfonate de styrène, l'alcool allylique et leurs mélanges.

Le monomère (a) peut contenir également des atomes de silicium, de fluor ou encore des groupements thio. Les monomères (a) peuvent être neutralisés s'ils contiennent des groupement acides, et ceci avant ou après la polymérisation, totalement ou partiellement, de façon à ajuster la solubilité ou le degré de dispersion dans l'eau au niveau désiré. En tant qu'agent servant pour la neutralisation, on peut utiliser les bases minérales, telles que le carbonate de sodium, les base organiques telles que les aminoalcools, comme les alcanolamines telles que la méthanolamine, le 2-amino-2-méthyl-1-propanol, la triéthanolamine et les diamines, comme la lysine.

Les monomères (a) peuvent être aussi quaternisés lorsquils possèdent un atome d'azote basique. S'ils possèdent au moins deux doubles liaisons éthyléniques, les monomères (a) peuvent être partiellement réticulés.

Les dérivés siliconés (b) sont notamment les composés connus, sous la dénomination INCI par diméthicone copolyols ou les tensioactifs siliconés. On peut citer ceux commercialisés sous la marque Abil ® par Goldschmidt, Alkasil ® par Rhône-Poulenc, silicone Polyol Copolymer ® par Genesee, Besil ® par Wacker, Silwet ® par OSI ou Dow Corning 190® par Dow Corning.

Parmi les monomères (b), on préfère ceux qui présentent la formule I ci-après : 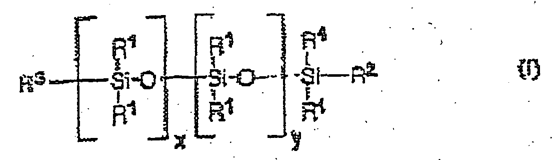 dans laquelle formule (I):
- R² est choisi parmi CH₃ ou le groupement 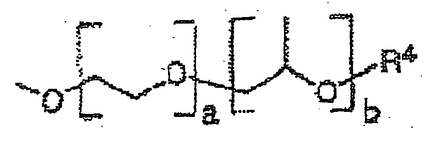
- R³ est choisi parmi CH₃ ou le groupement R²,
- R⁴ est choisi parmi l'hydrogène, CH₃, ou les groupements 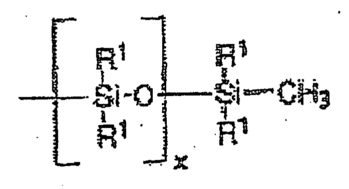 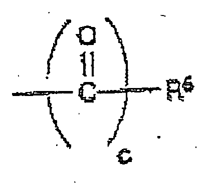
- R⁶ est un groupement organique en C₁ à C₄₀ pouvant contenir des groupements amino, carboxyles ou sulfonates, et si c=0, R₆ est l'anion d'un acide inorganique ;
- les radicaux R¹ peuvent être identiques ou différents et sont choisis parmi les hydrocarbures aliphatiques en C₁ à C₂₀, les hydrocarbures aliphatiques ou cycloaliphatiques en C₃ à C₂₀, et les groupements R⁵ répondant à la formule ci-après : 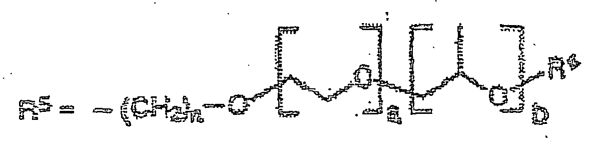
- n est un nombre entier compris entre 1 et 6,
- x et y sont des nombres entiers choisis de telle sorte que le poids moléculaire du polysiloxane soit compris entre 300 et 30 000,
- a et b sont des nombres entiers compris entre 0 et 50, et
- c est 0 ou 1.

De préférence, R¹ est choisi parmi les groupements méthyl, éthyl, propyl, butyl, isobutyl, pentyl, isopentyl, hexyl, octyl, décyl, dodécyl and octadécyl, les radicaux cycloaliphatiques, en particulier les groupements cyclohexyls, les groupements aromatiques, en particulier les groupements phényls ou naphthyls, les mélanges de radicaux aromatiques et aliphatiques, tels que le benzyl et le phenyléthyl, et aussi le tolyl and le xylyl.

On préfère les radicaux R⁴ ayant pour formule -(CO)c-R⁶, R⁶ étant un alkyl, cycloalkyl or aryl ayant 1 à 40 atomes de carbone qui peut contenir des groupements supplémentaires tels que NH₂, COOH et/ou SO₃H.

On préfère les radicaux R⁶ pour lesquels c=0 qui sont des phosphates ou des sulfates.

Les dérivés siliconés (b) particulièrement préférés sont ceux présentant la formule générale suivante :

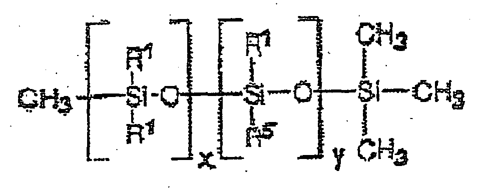

La proportion relative de dérivé (b) dans le copolymère est généralement de 0,1 à 50, et de préférence de 1 à 20 % en poids.

On préfère les copolymères silicone/acrylate solubles dans l'eau ou ceux dont la dispersibilité dans l'eau est telle que dans un mélange eau/éthanol dosé à 50 :50 en volume, ils sont solubles dans une proportion supérieure à 0,1 g/l, et de préférence supérieure à 0,2 g/l.

La composition comprend avantageusement, en pourcentage relatif en poids de la composition, de 0,1 à 20 % de copolymère silicone/acrylate, et plus préférentiellement de 0,5 à 10 % de ce copolymère.

La composition comprend avantageusement, en pourcentage relatif en poids de la composition, de 0,01 à 20 % d'agent de conditionnement et de préférence, de 0,05 à 10 %.

On peut citer comme silicones non volatiles, conformes à la présente invention, comportant dans leur structure générale, un ou plusieurs groupements aminés substitués ou non, directement fixés sur la chaîne siloxanique ou fixés par l'intermédiaire d'un radical hydrocarboné, la "GP 4 SILICONE FLUID®" de GENESEE, la "GP 7100®" de GENESEE, la "Q2 8220®" de Dow CORNING, l'"AFL 40®" d'UNION CARBIDE ou la silicone dénommée "Amodiméthicone" dans le dictionnaire CTFA.

Les viscosités des silicones peuvent être notamment déterminées par la norme ASTM D445-97 (viscométrie).

Parmi les alcools, on peut citer l'éthanol, l'isopropanol. L'éthanol est particulièrement préféré.

La composition de l'invention peut également contenir au moins un additif choisi parmi les tensioactifs autres que ceux de l'invention, les parfums, les filtres, les conservateurs, les protéines, les vitamines, les polymères autres que ceux de l'invention, les huiles minérales, végétales ou synthétiques autres que celles de l'invention, les agents épaississants et tout autre additif classiquement utilisé dans les compositions cosmétiques.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Ces compositions peuvent être conditionnées sous diverses formes, notamment dans des flacons pompes ou dans des récipients aérosols, afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux. Les compositions conformes à l'invention peuvent aussi se présenter sous la forme de crèmes, de gels, d'émulsions, de lotions ou de cires.

Lorsque la composition selon l'invention est conditionnée sous forme d'aérosol en vue d'obtenir une laque ou une mousse, elle comprend au moins un agent propulseur qui peut être choisi parmi les hydrocarbures volatils tels que le n-butane, le propane, l'isobutane, le pentane, un hydrocarbure halogéné et leurs mélanges. On peut également utiliser en tant qu'agent propulseur le gaz carbonique, le protoxyde d'azote, le diméthyléther (DME), l'azote, l'air comprimé. On peut aussi utiliser des mélanges de propulseurs. De préférence, on utilise le diméthyl éther.

Avantageusement, l'agent propulseur est présent à une concentration comprise entre 5 et 90 % en poids par rapport au poids total de la composition dans le dispositif aérosol et, plus particulièrement, à une concentration comprise entre 10 et 60 %.

Les compositions conformes à l'invention peuvent être appliquées sur la peau, les ongles, les lèvres, les cheveux, les sourcils et les cils.

Les compositions conformes à l'invention sont particulièrement adaptées pour être appliquées sur des cheveux secs ou humides, en tant que produit de coiffage.

## Revendications

1. Composition cosmétique comprenant, dans un milieu cosmétiquement acceptable, constitué par des alcools en C₁-C₄ ou par des mélanges eau-alcools en C₁-C₄ au moins un copolymère silicone/acrylate et au moins un agent de conditionnement, **caractérisée par le fait que**
- le copolymère silicone/acrylate est obtenu par polymérisation radicalaire d'au moins un monomère éthyléniquement insaturé (a) en présence d'au moins un dérivé siliconé (b) comprenant des groupements oxyalkylénés, et
- l'agent de conditionnement est une silicone non volatile comprenant des groupements aminés substitués ou non directement fixés sur la chaîne siloxanique ou fixés par l'intermédiaire d'un radical hydrocarboné.

2. Composition cosmétique selon la revendication 1, **caractérisée par le fait que** le monomère (a) répond à la formule (Iₐ) : 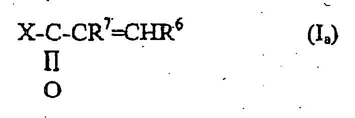 dans laquelle :
- X est choisi dans le groupe comprenant OH, OM, OR⁸, NH₂, NHR⁸, N(R⁸)₂ ;
- M est un cation choisi parmi Na⁺, K⁺, Mg⁺⁺, NH⁴⁺, alkylammonium, dialkylammonium, trialkylammonium, et tétraalkylammonium ;
- les radicaux R⁸ peuvent être identiques ou différents et sont choisis dans le groupe comprenant l'hydrogène, les groupements alkyls linéaires ou ramifiés en C₁ à C₄₀, les groupements alkyls en C₁ à C₄₀ mono- ou polyhydroxylés, éventuellement substitués par un ou plusieurs groupement(s) alcoxy, amino ou carboxy, les groupements polyéthers hydroxylés, les groupements N,N-diméthylaminoéthyl, 2-hydroxyéthyl, 2-méthoxyéthyl, 2-éthoxyéthyl, hydroxypropyl, méthoxypropyl et éthoxypropyl ;
- R⁷ et R⁶ sont choisis indépendamment l'un de l'autre dans le groupe comprenant l'hydrogène, les groupements alkyls linéaires ou ramifiés en C₁ à C₈, méthoxy, éthoxy, 2-hydroxyéthoxy, 2-méthoxyéthoxy et 2-éthoxyéthyl, les groupements CN, COOH et COOM.

3. Composition cosmétique selon la revendication t, **caractérisée par le fait que** le dérivé siliconé (b) répond à la formule I ci-après : 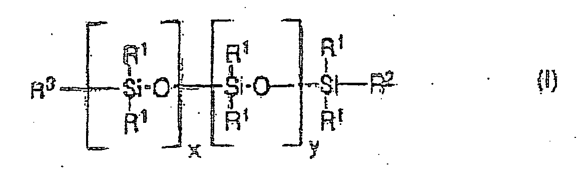 dans laquelle formule (I):
- R² est choisi parmi CH₃ ou le groupement 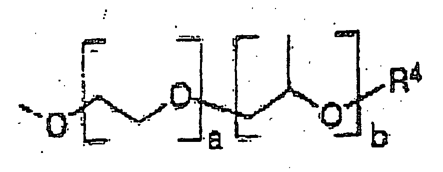
- R³ est choisi parmi CH₃ ou le groupement R²,
- R⁴ est choisi parmi l'hydrogène, CH₃, ou les groupements 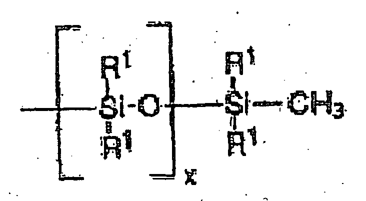 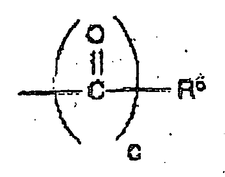
- R⁶ est un groupement organique en C₁ à C₄₀ pouvant contenir des groupements amino, carboxyles ou sulfonates, et si c=0, R₆ est l'anion d'un acide inorganique ;
- les radicaux R¹ peuvent être identiques ou différents et sont choisis parmi les hydrocarbures aliphatiques en C₁ à C₂₀, les hydrocarbures aliphatiques ou cycloaliphatiques en C₃ à C₂₀, et les groupements R⁵ répondant à la formule ci-après : 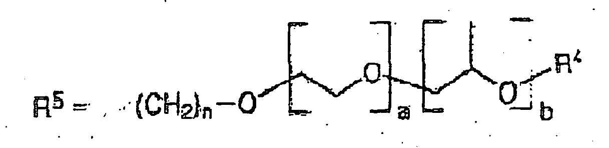
- n est un nombre entier compris entre 1 et 6,
- x et y sont des nombres entiers choisis de telle sorte que le poids moléculaire du polysiloxane soit compris entre 300 et 30 000,
- a et b sont des nombres entiers compris entre 0 et 50, et
- c est 0 ou 1.

4. Composition selon la revendication 1, **caractérisée par le fait que** le monomère (a) est choisi parmi les esters de vinyle et d'allyle en C₁ à C₄₀, les acides carboxyliques, linéaires en C₃ à C₄₀, ou carboxycycliques en C₃ à C₄₀, les halides de vinyle ou d'allyle, les vinyllactames, de préférence la vinylpyrrolidone et le vinylcaprolactame, les composés hétérocycles substitués par des groupement vinyles ou allyles, de préférence la vinylpyridine, la vinyloxazoline et l'allylpyridine, les N-vinylimidazoles, les diallylamines, le chlorure de vinylidène, les composés insaturés carbonés, comme le styrène ou l'isoprène, les dérivés quatemisés par l'épichlorhydrine de l'acide acrylique ou méthacrylique.

5. Composition selon la revendication 1, **caractérisée par le fait que** le monomère (a) est choisi dans le groupe comprenant l'acide acrylique, méthacrylique et éthylacrylique ; le méthyl, éthyl, propyl, n-butyl, isobutyl, t-butyl, 2-éthylhexyl et décyl acrylate ; le méthyl, éthyl, propyl, n-butyl, isobutyl, t-butyl, 2-éthylhexyl et décyl méthacrylate ; le méthyl, éthyl, propyl, n-butyl; isobutyl, t-butyl, 2-éthylhexyl et décyl éthacrylate ; le 2,3-dihydroxypropyl acrylate ; le 2,3-dihydroxypropyl méthacrylate ; le 2-dihydroxyéthyl acrylate ; l'hydroxypropyl acrylate ; le 2-hydroxyéthyl méthacrylate ; le 2-hydroxyéthyl éthacrylate ; le 2-méthoxyéthyl acrylate ; le 2-éthoxyéthyl méthacrylate ; le 2-éthoxyéthyl éthacrylate ; l'hydroxypropyl méthacrylate ; le glycéryl monoacrylate ; le glycéryl monométhacrylate ; les polyalkylènes glycols(méth-)acrylate, les acides sulfoniques insaturés, l'acrylamide, la méthacrylamide, l'éthacrylamide, la N,N-diméthylacrylamide, la N-éthylacrylamide, la N-éthylméthacrylamide, 1-vinylimidazole, N,N-diméthylaminoéthyl(méth-)acrylate, l'acide maléique, l'acide fumarique, l'anhydride maléique et ses monoesters, l'acide crotonique, l'acide itaconique, les éthers de vinyle, le vinylformamide, la vinylamine, la vinylpyridine, la vinylimidazole, le vinylfurane, le styrène, le sulfonate de styrène, l'alcool allylique et leurs mélanges.

6. Composition selon l'une quelconque des revendications précédentes, **caractérisée par le fait que** les dérivés siliconés (b) sont choisis parmi les diméthicone copolyols ou les tensioactifs siliconés.

7. Composition selon l'une quelconque des revendications précédentes, **caractérisée par le fait qu**'elle comprend, en pourcentage relatif en poids, de 0,1 à 20 % de copolymère silicone/acrylate, et plus préférentiellement de 0,5 à 10 % de ce copolymère.

8. Composition selon l'une quelconque des revendications précédentes, **caractérisée par le fait qu**'elle comprend, en pourcentage relatif en poids, de 0,01 à 20 % d'agent de conditionnement et de préférence, de 0,05 à 10 %.

9. Composition selon l'une quelconque des revendications précédentes, **caractérisée par le fait qu'**elle contient en outre au moins un additif choisi parmi les tensioactifs autres que ceux de l'invention, les parfums, les filtres, les conservateurs, les protéines, les vitamines, les polymères autres que ceux de l'invention, les huiles minérales, végétales ou synthétiques autres que celles de l'invention, les épaississants.

10. Dispositif aérosol formé par un récipient contenant une composition selon l'une quelconque des revendications précédentes et un gaz propulseur, ainsi qu'un moyen de distribution de la composition.

11. Procédé cosmétique, **caractérisé par le fait qu**'il comprend l'application d'une composition conforme à l'une quelconque des revendications 1 à 9, en particulier sur les cheveux.

12. Utilisation d'une composition selon l'une quelconque des revendications 1 à 9, pour la fabrication d'un produit cosmétique, en particulier capillaire,

13. Utilisation d'une composition selon l'une quelconque des revendications 1 à 9 pour les cheveux, les sourcils et les cils.

## Claims

1. Cosmetic composition comprising, in a cosmetically acceptable medium constituted of C₁-C₄ alcohols or mixtures of water and C₁-C₄ alcohols, at least one silicone/acrylate copolymer and at least one conditioning agent, **characterized in that**
- the silicone/acrylate copolymer is obtained by radical-mediated polymerization of at least one ethylenically unsaturated monomer (a) in the presence of at least one silicone derivative (b) comprising oxyalkylene groups, and
- the conditioning agent is a nonvolatile silicone comprising substituted or unsubstituted amino groups directly attached to the siloxane chain or attached via a hydrocarbon-based radical.

2. Cosmetic composition according to Claim 1, **characterized in that** the monomer (a) corresponds to formula (Iₐ): 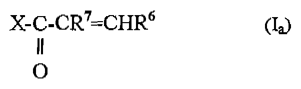 in which:
- X is chosen from the group comprising OH, OM, OR⁸, NH₂, NHR⁸ and N(R⁸)₂;
- M is a cation chosen from Na⁺, K⁺, Mg⁺⁺, NH⁴⁺, alkylammonium, dialkylammonium, trialkylammonium and tetraalkylammonium;
- the radicals R⁸ may be identical or different and are chosen from the group comprising hydrogen, linear or branched C₁ to C₄₀ alkyl groups, mono- or polyhydroxylated C₁ to C₄₀ alkyl groups, optionally substituted with one or more alkoxy, amino or carboxyl groups, hydroxylated polyether groups, and N,N-dimethylaminoethyl, 2-hydroxyethyl, 2-methoxyethyl, 2-ethoxyethyl, hydroxypropyl, methoxypropyl and ethoxypropyl groups;
- R⁷ and R⁶ are chosen, independently of each other, from the group comprising hydrogen, linear or branched C₁ to C₈ alkyl groups, methoxy, ethoxy, 2-hydroxyethoxy, 2-methoxyethoxy and 2-ethoxyethyl groups, and CN, COOH and COOM groups.

3. Cosmetic composition according to Claim 1, **characterized in that** the silicone derivative (b) corresponds to the formula (I) below: 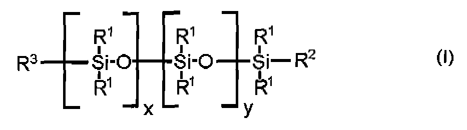 in which formula (I):
- R² is chosen from CH₃ and the group 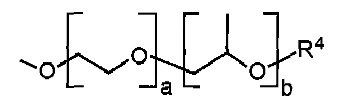
- R³ is chosen from CH₃ and the group R²,
- R⁴ is chosen from hydrogen, CH₃ and the groups 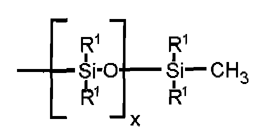 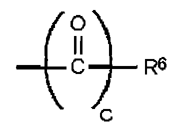
- R⁶ is an organic C₁ to C₄₀ group which can contain amino, carboxyl or sulfonate groups, and if c=0, R₆ is the anion of an inorganic acid;
- the radicals R¹ may be identical or different and are chosen from C₁ to C₂₀ aliphatic hydrocarbons, C₃ to C₂₀ aliphatic or cycloaliphatic hydrocarbons, and the groups R⁵ corresponding to the formula below: 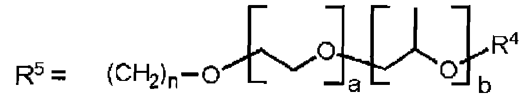
- n is an integer between 1 and 6,
- x and y are integers chosen such that the molecular weight of the polysiloxane is between 300 and 30,000,
- a and b are integers between 0 and 50, and
- c is 0 or 1.

4. Composition according to Claim 1, **characterized in that** the monomer (a) is chosen from C₁ to C₄₀ vinyl and allyl esters, linear C₃ to C₄₀ carboxylic acids or C₃ to C₄₀ carboxycyclic acids, vinyl or allyl halides, vinyllactams, preferably vinylpyrrolidone and vinylcaprolactam, heterocyclic compounds substituted with vinyl or allyl groups, preferably vinylpyridine, vinyloxazoline and allylpyridine, N-vinylimidazoles, diallylamines, vinylidene chloride, carbon-based unsaturated compounds, such as styrene or isoprene, and acrylic or methacrylic acid derivatives quaternized with epichlorohydrin.

5. Composition according to Claim 1, **characterized in that** the monomer (a) is chosen from the group comprising acrylic, methacrylic and ethacrylic acid; methyl, ethyl, propyl, n-butyl, isobutyl, t-butyl, 2-ethylhexyl and decyl acrylate; methyl, ethyl, propyl, n-butyl, isobutyl, t-butyl, 2-ethylhexyl and decyl methacrylate; methyl, ethyl, propyl, n-butyl, isobutyl, t-butyl, 2-ethylhexyl and decyl ethacrylate; 2,3-dihydroxypropyl acrylate; 2,3-dihydroxypropyl methacrylate; 2-dihydroxyethyl acrylate; hydroxypropyl acrylate; 2-hydroxyethyl methacrylate; 2-hydroxyethyl ethacrylate; 2-methoxyethyl acrylate; 2-ethoxyethyl methacrylate; 2-ethoxyethyl ethacrylate; hydroxypropyl methacrylate; glyceryl monoacrylate; glyceryl monomethacrylate; polyalkylene glycol (meth)acrylates, unsaturated sulfonic acids, acrylamide, methacrylamide, ethacrylamide, N,N-dimethylacrylamide, N-ethylacrylamide, N-ethylmethacrylamide, 1-vinylimidazole, N,N-dimethylaminoethyl (meth)acrylate, maleic acid, fumaric acid, maleic anhydride and its monoesters, crotonic acid, itaconic acid, vinyl ethers, vinylformamide, vinylamine, vinylpyridine, vinylimidazole, vinylfuran, styrene, styryl sulfonate and allyl alcohol, and mixtures thereof.

6. Composition according to any one of the preceding claims, **characterized in that** the silicone derivatives (b) are chosen from dimethicone copolyols and silicone surfactants.

7. Composition according to any one of the preceding claims, **characterized in that** it comprises, as a relative percentage by weight, from 0.1% to 20% of silicone/acrylate copolymer, and more preferably from 0.5% to 10% of this copolymer.

8. Composition according to any one of the preceding claims, **characterized in that** it comprises, as a relative percentage by weight, from 0.01% to 20% of conditioning agent and preferably from 0.05% to 10%.

9. Composition according to any one of the preceding claims, **characterized in that** it also contains at least one additive chosen from surfactants other than those of the invention, fragrances, screening agents, preserving agents, proteins, vitamins, polymers other than those of the invention, mineral, plant or synthetic oils other than those of the invention, and thickeners.

10. Aerosol device formed from a container containing a composition according to any one of the preceding claims and a propellent gas, and also a means for distributing the composition.

11. Cosmetic process, **characterized in that** it comprises the application of a composition in accordance with any one of Claims 1 to 9, in particular to the hair.

12. Use of a composition according to any one of Claims 1 to 9, for the manufacture of a cosmetic product, in particular a hair product.

13. Use of a composition according to any one of Claims 1 to 9, for the hair, the eyebrows and the eyelashes.

## Patentansprüche

1. Kosmetische Zusammensetzung, die in einem kosmetisch akzeptablen Medium, das aus Alkoholen mit 1 bis 4 Kohlenstoffatomen oder Gemischen von Wasser und Alkoholen mit 1 bis 4 Kohlenstoffatomen besteht, mindestens ein Silicon/Acrylat-Copolymer und mindestens ein Konditioniermittel enthält, **dadurch gekennzeichnet, dass**
- das Silicon/Acrylat-Copolymer durch radikalische Polymerisation mindestens eines ethylenisch ungesättigten Monomers (a) in Gegenwart mindestens eines Siliconderivats (b) mit Alkylenoxidgruppen hergestellt wird, und
- das Konditioniermittel ein nicht flüchtiges Silicon mit substituierten oder unsubstituierten aminierten Gruppen ist, die an die Siloxankette direkt oder über eine Kohlenwasserstoffgruppe gebunden sind.

2. Kosmetische Zusammensetzung nach Anspruch 1, **dadurch gekennzeichnet, dass** das Monomer (a) der folgenden Formel (Ia) entspricht: 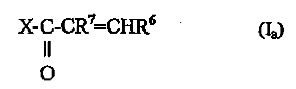 worin:
- X unter OH, OM, OR⁸, NH₂, NHR⁸, N(R⁸)₂ ausgewählt ist;
- M ein Kation bedeutet, das unter Na⁺, K⁺, Mg⁺⁺, NH₄⁺, Alkylammonium, Dialkylammonium, Trialkylammonium und Tetraalkylammonium ausgewählt ist;
- die Gruppen R⁸ identisch oder voneinander verschieden sein können und unter Wasserstoff, geradkettigen oder verzweigten C₁₋₄₀-Alkylgruppen, mono- oder polyhydroxylierten C₁₋₄₀-Alkylgruppen, die ggf. mit einer oder mehreren Gruppen Alkoxy, Amino oder Carboxy substituiert sind, hydroxylierten Polyethergruppen, N,N-Dimethylaminoethyl, 2-Hydroxyethyl, 2-Methoxyethyl, 2-Ethoxyethyl, Hydroxypropyl, Methoxypropyl und Ethoxypropyl ausgewählt sind;
- die Gruppen R⁷ und R⁶ unabhängig voneinander unter Wasserstoff, geradkettigen oder verzweigten C₁₋₈-Alkylgruppen, Methoxy, Ethoxy, 2-Hydroxyethoxy, 2-Methoxyethoxy et 2-Ethoxyethyl und den Gruppen CN, COOH und COOM ausgewählt sind.

3. Kosmetische Zusammensetzung nach Anspruch 1, **dadurch gekennzeichnet, dass** das Siliconderivat (b) der folgenden Formel (I) entspricht: 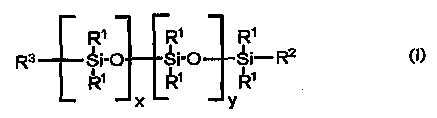 wobei in der Formel (I):
- R² ausgewählt ist unter CH₃ oder der Gruppe 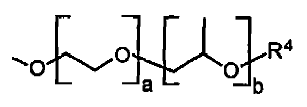
- R³ unter CH₃ oder der Gruppe R² ausgewählt ist,
- R⁴ ausgewählt ist unter Wasserstoff, CH₃ oder den folgenden Gruppen 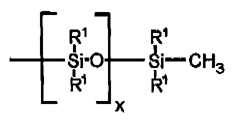 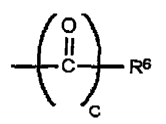
- R⁶ eine organische Gruppe mit 1 bis 40 Kohlenstoffatomen ist, die Amino-, Carboxy- oder Sulfatgruppen enthalten kann, und R₆ das Anion einer anorganischen Säure ist, wenn c=0;
- die Gruppen R¹ identisch oder verschieden sein können und unter den aliphatischen C₁₋₂₀-Kohlenwasserstoffen, aliphatischen oder cycloaliphatischen C₃₋₂₀-Kohlenwasserstoffen und den Gruppen R⁵ der folgenden Formel ausgewählt sind: 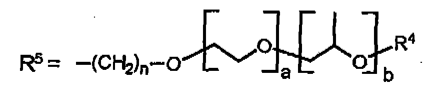
- n eine ganze Zahl im Bereich von 1 bis 6 bedeutet,
- x und y ganze Zahlen sind, die so ausgewählt sind, dass die Molmasse des Polysiloxans im Bereich von 300 bis 30 000 liegt,
- a und b 0 bedeuten oder ganze Zahlen im Bereich von 1 bis 50 sind, und
- c 0 oder 1 bedeutet.

4. Zusammensetzung nach Anspruch 1, **dadurch gekennzeichnet, dass** das Monomer (a) unter den Vinyl- und Allylestern mit 1 bis 40 Kohlenstoffatomen, geradkettigen Carbonsäuren mit 3 bis 40 Kohlenstoffatomen oder carboxycyclischen Carbonsäuren mit 3 bis 40 Kohlenstoffatomen, Vinyl- oder Allylhaliden, Vinyllactamen, vorzugsweise Vinylpyrrolidon und Vinylcaprolactam, mit Vinyl- oder Allylgruppen substituierten heterocyclischen Verbindungen, vorzugsweise Vinylpyridin, Vinyloxazolin und Allylpyridin, N-Vinylimidazolen, Diallylaminen, Vinylidenchlorid, ungesättigten Verbindungen auf Kohlenstoffbasis, wie Styrol oder Isopren, und mit Epichlorhydrin quaternisierten Derivaten von Acrylsäure oder Methacrylsäure ausgewählt ist.

5. Zusammensetzung nach Anspruch 1, **dadurch gekennzeichnet, dass** das Monomer (a) ausgewählt ist unter: Acrylsäure, Methacrylsäure und Ethacrylsäure; Methylacrylat, Ethylacrylat, Propylacrylat, n-Butylacrylat, Isobutylacrylat, *t*-Butylacrylat, 2-Ethylhexylacrylat und Decylacrylat; Methylmethacrylat, Ethylmethacrylat, Propylmethacrylat, n-Butylmethacrylat, Isobutylmethacrylat, *t-*Butylmethacrylat, 2-Ethylhexylmethacrylat und Decylmethacrylat; Methylethacrylat, Ethylethacrylat, Propylethacrylat, n-Butylethacrylat, Isobutylethacrylat, *t*-Butylethacrylat, 2-Ethylhexylethacrylat und Decylethacrylat; 2,3-Dihydroxypropylacrylat; 2,3-Dihydroxypropylmethacrylat; 2-Dihydroxyethylacrylat; Hydroxypropylacrylat; 2-Hydroxyethylmethacrylat; 2-Hydroxyethylethacrylat; 2-Methoxyethylacrylat; 2-Ethoxyethylmethacrylat; 2-Ethoxyethylethacrylat; Hydroxypropylmethacrylat; Glycerylmonoacrylat; Glycerylmonomethacrylat; Polyalkylenglykol(meth)acrylaten, ungesättigten Sulfonsäuren, Acrylamid, Methacrylamid, Ethacrylamid, N,N-Dimethylacrylamid, N-Ethylacrylamid, N-Ethylmethacrylamid, 1-Vinylimidazol, N,N-Dimethylaminoethyl(meth)acrylat, Maleinsäure, Fumarsäure, Maleinsäureanhydrid und ihren Monoestern, Crotonsäure, Itaconsäure, Vinylethern, Vinylformamid, Vinylamin, Vinylpyridin, Vinylimidazol, Vinylfuran, Styrol, Styrolsulfonat, Allylalkohol und deren Gemischen.

6. Zusammensetzung nach einem der vorhergehenden Ansprüche, **dadurch gekennzeichnet, dass** die Siliconderivate (b) unter Dimethiconcopolyolen oder siliconhaltigen grenzflächenaktiven Stoffen ausgewählt sind.

7. Zusammensetzung nach einem der vorhergehenden Ansprüche, **dadurch gekennzeichnet, dass** sie 0,1 bis 20 Gew.-% Silicon/Acrylat-Copolymer und vorzugsweise 0,5 bis 10 Gew.-% Copolymer enthält.

8. Zusammensetzung nach einem der vorhergehenden Ansprüche, **dadurch gekennzeichnet, dass** das Konditioniermittel in der Zusammensetzung in einer Konzentration von 0,01 bis 20 Gew.-% und vorzugsweise 0,05 bis 10 Gew.-% enthalten ist.

9. Zusammensetzung nach einem der vorhergehenden Ansprüche, **dadurch gekennzeichnet, dass** sie ferner mindestens einen Zusatzstoff enthält, der unter grenzflächenaktiven Stoffen, die von den erfindungsgemäßen grenzflächenaktiven Stoffen verschieden sind, Parfums, Filtern, Konservierungsmitteln, Proteinen, Vitaminen, Polymeren, die von den erfindungsgemäßen Polymeren verschieden sind, pflanzlichen, mineralischen oder synthetischen Ölen, die von den erfindungsgemäßen Ölen verschieden sind, und Verdickungsmitteln ausgewählt ist.

10. Aerosolvorrichtung, die aus einem Behälter, der eine Zusammensetzung nach einem der vorhergehenden Ansprüche und ein Treibmittel enthält, und Einrichtungen zur Abgabe der Zusammensetzung besteht.

11. Kosmetisches Verfahren, **dadurch gekennzeichnet, dass** eine Zusammensetzung nach einem der Ansprüche 1 bis 9 insbesondere auf die Haare aufgetragen wird.

12. Verwendung einer Zusammensetzung nach einem der Ansprüche 1 bis 9 zur Herstellung eines kosmetischen Produkts und insbesondere eines kosmetischen Produkts insbesondere für die Haarbehandlung.

13. Verwendung einer Zusammensetzung nach einem der Ansprüche 1 bis 9 für die Haare, die Wimpern und die Augenbrauen.
